# EUROPEAN PATENT SPECIFICATION

(11) **EP 1 934 608 B1**
(45) Date of publication and mention of the grant of the patent: **15.03.2017**
(21) Application number: 06824918.4
(22) Date of filing: 08.09.2006
(51) Int. Cl.: G06F 7/00, G01N 33/50, G01N 33/564, G01N 33/68, G01N 33/574

(54) **TARGETED IDENTIFICATION OF IMMUNOGENIC PEPTIDES**
GEZIELTE IDENTIFIKATION IMMUNOGENER PEPTIDE
IDENTIFICATION CIBLÉE DE PEPTIDES IMMUNOGÈNES

(30) Priority: 08.09.2005 US 714865 P
(43) Date of publication of application: 25.06.2008
(73) Proprietor: HENRY M. JACKSON FOUNDATION FOR THE ADVANCEMENT OF MILITARY MEDICINE, INC., Bethesda, MD 20817 (US)
(72) Inventor: PONNIAH, Sathibalan, Columbia, MD 21044 (US); PEOPLES, George, E., Fulton, MD 20759 (US); STORRER, Catherine, E., Columbia, MD 21044 (US); FLORA, Michael, Mt. Airy, MD 21771 (US)
(74) Representative: Steinecke, Peter
(86) International application number: PCT/US2006/035171
(87) International publication number: WO 2007/030771

(56) References cited:
- WO-A1-94/20127
- US-A- 5 726 023
- US-A1- 2004 018 971
- US-B2- 7 179 645
- KNUTSON K L ET AL: "Tumor antigen-specific T helper cells in cancer immunity and immunotherapy", CANCER IMMUNOLOGY, IMMUNOTHERAPY, SPRINGER, BERLIN, DE, vol. 54, no. 8, 1 August 2005 (2005-08-01) , pages 721-728, XP019333158, ISSN: 1432-0851, DOI: 10.1007/S00262-004-0653-2
- ABRAHAM MITTELMAN ET AL: "Monoclonal and polyclonal humoral immune response to EC HER-2/NEU peptides with low similarity to the host's proteome", INTERNATIONAL JOURNAL OF CANCER, vol. 98, no. 5, 10 April 2002 (2002-04-10) , pages 741-747, XP055111438, ISSN: 0020-7136, DOI: 10.1002/ijc.10259
- DAKAPPAGARI N K ET AL: "Prevention of mammary tumors with a chimeric HER-2 B-cell epitope peptide vaccine", CANCER RESEARCH, AMERICAN ASSOCIATION FOR CANCER RESEARCH, US, vol. 60, no. 14, 15 July 2000 (2000-07-15) , pages 3782-3789, XP002290184, ISSN: 0008-5472
- RAMASAMY R ET AL: "Characterisation of an inhibitory monoclonal antibody-defined epitope on a malaria vaccine candidate antigen", IMMUNOLOGY LETTERS, ELSEVIER BV, NL, vol. 23, no. 4, 1 February 1990 (1990-02-01), pages 305-309, XP023668953, ISSN: 0165-2478, DOI: 10.1016/0165-2478(90)90077-4 [retrieved on 1990-02-01]
- WAGNER C ET AL: "Identification of an HLA-A*02 restricted immunogenic peptide derived from the cancer testis antigen HOM-MEL-40/SSX2", CANCER IMMUNITY, ACADEMY OF CANCER IMMUNOLOGY, CH, vol. 3, no. 18, 1 December 2003 (2003-12-01), pages 1-15, XP002486698, ISSN: 1424-9634
- N. K. Dakappagari: "Conformational HER-2/neu B-cell epitope peptide vaccine designed to incorporate two native disulfide bonds enhances tumor cell binding and antitumor activities", Journal of Biological Chemistry, vol. 280, no. 1, 1 January 2004 (2004-01-01), pages 54-63, XP055047782, US ISSN: 0021-9258, DOI: 10.1074/jbc.M411020200
- ZUM BUSCHENFELDE C M ET AL: "Antihuman Epidermal Growth Factor Receptor 2 (HER2) monoclonal antibody trastuzumab enhances cytolytic activity of class I-restricted HER2-specific T lymphocytes against HER2-overexpressing tumor cells", CANCER RESEARCH, AMERICAN ASSOCIATION FOR CANCER RESEARCH, US, vol. 62, no. 8, 15 April 2002 (2002-04-15) , pages 2244-2247, XP002981803, ISSN: 0008-5472
- KONO K ET AL: "Trastuzumab (Herceptin) Enhances Class I-restricted antigen presentation Recognized by HER-2/neu-specific T Cytotoxic Lymphocytes", CLINICAL CANCER RESEARCH, THE AMERICAN ASSOCIATION FOR CANCER RESEARCH, US, vol. 10, no. 7, 1 April 2004 (2004-04-01), pages 2538-2544, XP002981804, ISSN: 1078-0432, DOI: 10.1158/1078-0432.CCR-03-0424

## Description

### Reference to Related Applications

This application claims priority to United States Provisional Application No. 60/714,865 entitled "*Targeted Identification of Immunogenic Peptides*" filed September 8, 2005.

### Background of the Invention

### 1. Field of the Invention

This invention, in the field of immunology/immunotherapy, vaccine discovery and development, relates generally to the identification of immunogenic peptides from regions of proteins and molecules that are involved in the binding interactions with polyclonal and monoclonal antibodies. The present invention is directed to methods for identification and use of the peptides for preventing, suppressing and treating immune-related diseases. Specifically, the invention provides therapy that result in clinical improvement in cancer patients.

### 2. Description of the Background

Autoimmune diseases are characterized by an unwanted and unwarranted attack by the immune system on the tissues of the host. While the mechanism for progress of these diseases is not well understood, at least some of the details with respect to antigen presentation are known. It is thought that antigens, including autoantigens, are processed by antigen-presenting cells (APC), and the resulting fragments are then associated with one of the cell surface proteins encoded by the major histocompatibility complex (MHC). As a result, recognition of a peptide antigen is said to be MHC "restricted." When the MHC/antigen fragment complex binds to a complementary T cell receptor (TCR) on the surface of a T lymphocyte, activation and proliferation of the clone or subpopulation of T cells result bearing that particular TCR. Once activated, T cells have the capacity to regulate other cells of the immune system which display the processed antigen and to destroy the cells or tissues which carry epitopes of the recognized antigen.

Antibody therapies in which antibodies are directed to MHC molecules and CD4 molecules have been generally successful in several animal models of autoimmunity. However, these approaches may be too nonspecific and potentially overly suppressive. This may be because 70% of T cells bear the CD4 marker and because all T cell-mediated responses and most antibody responses require MHC-associated antigen presentation.

A major difficulty with present approaches is that they require the use of complex biological preparations which do not comprise well-defined therapeutic agents. Such preparations suffer from complex production and maintenance requirements (e.g., the need for sterility and large quantities of medium for producing large number of "vaccine" T cells), and lack reproducibility from batch to batch. To be useful in humans, T cell "vaccine" preparations must be both autologous and individually specific. This means they must be uniquely tailored for each patient. Furthermore, the presence of additional antigens on the surface of such T cells may result in a broader, possibly detrimental, immune response not limited to the desired T cell clones (Offner et al., J. Neuroimmunol. 21:13-22 (1989).

Kono et al. (Clin Cancer Res 10, 2538) discloses that trastuzumab enhances cytotoxic activity of CTLs against HER2-overexpressing tumors.

There is a need, therefore, for agents and pharmaceutical compositions which have the properties of specificity for the targeted immune response. These agents and compositions should also have predictability in their selection, convenience and reproducibility of preparation, and sufficient definition in order to permit precise control of dosage.

An effective vaccine is capable of generating a long-lasting immunity while being relatively harmless to the recipient. Attenuated organisms and purified antigens from organisms have traditionally been used as vaccines. However, such agents often produce deleterious side effects or fail to protect against subsequent challenges. Because of the inherent difficulties in growing pathogenic organisms and producing effective vaccines from them, many viral, bacterial and parasitic diseases have no effective vaccine.

A further difficulty with the use of peptides as vaccines is that, in most instances, peptides alone are not good immunogens. It is a well known phenomenon that most immune responses to peptide antigens are T cell-dependent. Accordingly, "carrier" molecules have been attached to peptide antigens that bind, for example, to B cell surface immunoglobulin in order to generate a high affinity, IgG response. In other words, nonresponsiveness to peptide antigens may sometimes be overcome by attaching another peptide that induces helper T cell activity.

In general, peptides that induce helper T cell activity are generated by B cells from enzymatic digestion of native proteins internalized by way of an antibody receptor. These T cell stimulating peptides are then presented on the surface of the B cell in association with class II major histocompatibility complex (MHC) molecules. In a similar fashion, peptides that induce cytotoxic T cell activity may be generated by accessory cells, including B cells. These peptides are presented on the cell surface of accessory cells in association with class I MHC molecules. As used herein, the term "T cell stimulatory peptide" means any peptide which activates or stimulates T cells, including (but not limited to) helper T cells and/or cytotoxic T cells.

Peptides represent a promising approach to the production and design of vaccines. However, the difficulties in making peptides that induce the desired immune response have hampered their success. This includes the difficulties inherent in making peptides that closely mimic the native structure of antigenic determinants.

These antigenic determinants, or epitopes, of a protein antigen represent the sites that are recognized as binding sites by certain immune components such as antibodies or immunocompetent cells. While epitopes are defined only in a functional sense, i.e. by their ability to bind to antibodies or immunocompetent cells, there is a structural basis for their immunological activity.

Epitopes are classified as either being continuous and discontinuous. Discontinuous epitopes are composed of sequences of amino acids throughout an antigen and rely on the tertiary structure or folding of the protein to bring the sequences together and form the epitope. In contrast, continuous epitopes are linear peptide fragments of the antigen that are able to bind to antibodies raised against the intact antigen.

Many antigens have been studied as possible serum markers for different types of cancer because the serum concentration of the specific antigen may be an indication of the cancer stage in an untreated person. As such, it would be advantageous to develop immunological reagents that react with the antigen. More specifically, it would be advantageous to develop immunological reagents that react with the epitopes of the protein antigen.

Conventional methods using biochemical and biophysical properties have attempted to determine the location of probable peptide epitopes. These methods include a careful screening of a protein's primary structure, searching for critical turns, helices, and even the folding of the protein in the tertiary structure. Continuous epitopes are structurally less complicated and therefore may be easier to locate. However, the ability to predict the location, length and potency of the site is limited.

Various other methods have been used to identify and predict the location of continuous epitopes in proteins by analyzing certain features of their primary structure. For example, parameters such as hydrophilicity, accessibility and mobility of short segments of polypeptide chains have been correlated with the location of epitopes.

Hydrophilicity has been used as the basis for determining protein epitopes by analyzing an amino acid sequence in order to find the point of greatest local hydrophilicity. As discussed in U.S. Pat. No. 4,554,101, each amino acid is assigned a relative hydrophilicity numerical value which is then averaged according to local hydrophilicity so that the locations of the highest local average hydrophilicity values represent the locations of the continuous epitopes. However, this method does not provide any information as to the optimal length of the continuous epitope. Similarly, U.S. Pat. No. 6,780,598 B1 determines the immunopotency of an epitope by providing a ranking system delineating between dominant and subdominant epitopes.

Computer-driven algorithms have been devised to take advantage of the biochemical properties of amino acids in a protein sequence by sorting information to search for T cell epitopes. These algorithms have been used to search the amino acid sequence of a given protein for characteristics known to be common to immunogenic peptides. They can often locate regions that are likely to induce cellular immune response *in vitro.* Computer-driven algorithms can identify regions of proteins that contain epitopes which are less variable among geographic isolates, or regions of each geographic isolate's more variable proteins, or perform as a preliminary tool to evaluate the evolution of immune response to an individual's own quasi species.

Peptides presented in conjunction with class I MHC molecules are derived from foreign or self protein antigens that have been synthesized in the cytoplasm. Peptides presented with class II MHC molecules are usually derived from exogenous protein antigens. Peptides binding to class I molecules are usually shorter (about 8-10 amino acid residues) than those that bind to class II molecules (8 to greater than 20 residues).

Identification of T cell epitopes within protein antigens has traditionally been accomplished using a variety of methods. These include the use of whole and fragmented native or recombinant antigenic protein, as well as the more commonly employed "overlapping peptide" method for the identification of T cell epitopes within protein antigens which involves the synthesis of overlapping peptides spanning the entire sequence of a given protein. Peptides are then tested for their capacity to stimulate T cell cytotoxic or proliferation responses *in vitro.*

The overlapping peptide method is both cost and labor intensive. For example, to perform an assay using 15 amino acid long peptides overlapping by 5 amino acids spanning a given antigen of length n (a small subset of the possible 15-mers spanning the protein), one would need to construct and assay (n/5)-1 peptides. For most types of analyses, this number would be prohibitive.

Accordingly, a simple method to identify immunogenic peptides from regions of self-proteins and other proteins and molecules involved in binding interactions with polyclonal and monoclonal antibodies is needed.

### Summary of the Invention

The invention overcomes the problems and disadvantages associated with current methods and provides tools and methods of generating an immune response in a patient in need thereof as characterized in the claims.

One embodiment of the method of the invention includes synthesizing an immunogenic peptide from a self-protein comprising the steps of identifying one or more peptide sequences of a self protein that are directly or indirectly involved with antibody-binding, subjecting the one or more peptide sequences to an algorithm that identifies sequences suspected of being immunogenic, screening all peptide fragments from the one or more peptide sequences, and identifying an immunogenic peptide of the protein fragment wherein the antibody-binding interaction is polyclonal or monoclonal. Further, a patient may be treated with the immunogenic peptide to generate an immune response.

Another embodiment of the method of the invention includes identifying a vaccine treatment comprising the steps of binding an antibody to the self-protein forming a complex, subjecting the complex to proteasome digestion, obtaining digestion products comprising peptides, and identifying an immunogenic peptide sequence from the digestion products.

The present application also discloses a method of identifying a patient-specific treatment comprising the steps of obtaining a pre-existing immuno-reactive precursor to said patient's AGIE/ABIE, culturing tumor cells obtained from said patient, incubating the cultured tumor cells that are reactive against generated antibodies, examining dataset responses in presence and absence of the generated antibodies and identifying the patient-specific immunogenic epitopes. This method may include the generation of antibodies that are reactive against self antigens, the generation of antibodies that are reactive against foreign antigens, and/or the generation of antibodies, once administered to said patient, that are therapeutic or prophylactic.

Other embodiments and technical advantages of the invention are set forth below and may be apparent from the drawings and the description of the invention which follow, or may be learned from the practice of the invention.

### Description of the Invention and Examples

Treatments for complex diseases involving the immune system and immune responses caused by endogenous self antigens and/or foreign antigens that are involved in producing autoimmune antibody are extremely difficult to discover. Antigens involved with such diseases are either foreign or self antigens (or both). Administration of foreign antigens for passive immunization can result in serum sickness-like immune complex diseases. Also, reactive T cells capable of recognizing self peptides are typically deleted or processed and destroyed. These peptides, generated and displayed under normal and constitutive conditions are degraded by cell protein degradation machinery resulting in the absence of an immune response.

A simple method for identification of immunogenic regions of self-proteins and other proteins and molecules involved in the binding interactions with polyclonal and monoclonal antibodies has been surprisingly discovered. From this method, new and unique epitopes are generated that are presented in the presence of bound antibodies. Once these immunogenic regions are identified, vaccines comprising the antigen, modifications of the antigen, or antibodies specifically reactive to the antigen or the modified antigen can be prepared. Thus, the present invention makes vaccine peptide discovery manageable, and allows for the specific generation of unique immunogenic peptides from self-tumor associated proteins that can be induced or generated for specific expression in the presence of the antibody, allowing for vaccine and/or novel combination therapy.

The invention is characterized in the claims and described more fully herein and refers to many preferred embodiments. This invention, however, should not be construed as limited to those embodiments.

The proteasome is a multi-subunit complex with proteolytic cleavage activities that result in the generation of a wide variety of peptides from proteins. The susceptibility of a given protein to the proteolytic activities of the proteasome is dependent upon the various primary, secondary and tertiary structural and post-translational modifications that take place within the proteasome. These activities expose certain sequences or regions of the protein and not others, to the system.

In one embodiment of the method of the invention, cancer cells are induced to express immunogenic peptides from unique or self tumor-specific antigens to stimulate anti-tumor immune responses for immunotherapy. Normally these peptides are not be generated from self-proteins. Binding of antibodies (or other molecules) to the sites normally accessible and processed by proteasomes alters the pattern of accessibility and the resulting proteolytic cleavage pattern by the proteasome. Such alteration of the site results in generation of novel peptides that may be intrinsically immunogenic because they have not been previously expressed and displayed for the deletion of immuno-reactive T cells.

In one preferred embodiment, a unique immunogenic region of the HER-2/neu was identified. HER-2/neu is an over-expressed oncogenic protein. Conventional vaccine strategies, normally effective in immunizing patients, did not work with a "self-protein" such as HER-2/neu. Tolerance to self-proteins may only be directed to dominant epitopes of the protein and not the entire protein. Therefore, immunization to just a specific protein fragment, and not the entire protein, alleviates this problem. This specific protein fragment is located with within the sequence directly involved with antibody-binding interaction or in the proximity of that region.

After identifying this restricted, shorter peptide sequence, the sequence is subjected to algorithms to identify likely functionally active or target sequences or regions. Running algorithms on this segment, as opposed to the whole segment, provides a manageable set of peptides to test as candidates for vaccine development. In the past, computer-driven tests were run on the entire sequence consuming much time, money and effort. The algorithms searched the amino acid sequence provided for characteristic immune response *in vitro.* Regions of the proteins identified as containing epitopes may be useful as a vaccine.

Next, treatment of the tumor cells with the antibody against the identified peptide sequence was performed. Inducibility of the altered turnover and subsequent generation of the newly identified peptides only in the tumor cells and only in the presence of the antibody gave it a specific targeting and triggering feature that was controllable. An antibody booster can be given to increase the peptide-specific cytotoxic T cell response. In cases where the antibody already existed, discovery of the peptide only was sufficient for triggering the specific immune response.

The method to generate new and unique epitopes included the identification of immunogenic peptides from regions of proteins and molecules involved in the binding interactions with most any ligand, in particular polyclonal and monoclonal antibodies. These new epitopes were presented to antigen-processing cells in the presence of bound ligand. In certain cases, binding protected the epitope or a portion of the epitope revealing the immunogenic portion of the antigen. These immunogenic regions become new and uniquely enhanced targets for recognition by the immune system and for use in modulation of the immune responses for the treatment of disease states and immunotherapy either by themselves, an antibody vaccines, or when used in combination with antibodies. These antibody-generated immunogenic epitopes (AGIE)/antibody-bound immunogenic epitopes (ABIE) were useful for stimulating immune response in cancer and infectious diseases. They can also suppress immune responses in autoimmune diseases and transplantation based upon the activity of the antibody in modulating the immune response in the direction ofupregulation or down regulation.

Peptides varying in length including single chain antigen-binding polypeptides that can specifically interact, protect and/or modulate any given epitope such that it results in specifically protecting and/or enhancing the preservation and presentation of the epitope when subjected to the various proteolytic activities of the antigen processing machinery of the proteasome and immunoproteasome subunits and complexes.

Another embodiment of this invention comprises a combination of specific antibodies and/or treatment with other molecules that involve the same regions of the peptide sequences and targets unique populations of cells. This technology allows for the development of novel vaccines made of antibodies and/or antigen-binding fragments Fab or F(ab)'2-fragments bound to specific length peptides that contain immunogenic epitopes(s) of interest that are specifically processed and presented.

Specific epitopes of proteins may be protected, both intracellular and extracellular, that are normally destroyed by antigen processing machinery of proteasomes and immunoproteasomes by single chain antibodies and/or peptides that can specifically bind to selected site sequences so that these epitopes become novel targets that are developed as unique vaccines for the specific recognition of cancer cells. HSPs that are released during inflammatory processes perform similar activities and enhance immune responses because they were made to bind to many different peptide sequences non-covalently.

The method of the invention includes the identification of highly immunogenic peptides. These peptides are used to enhance or suppress immune responses from normal cell proteins that are not processed and presented naturally because of the constitutive destruction of these epitopes by the normal activities of the proteasome and immunoproteasome complexes. These peptides are used for generation for specific antibodies that bind and generate the novel presentation of these epitopes for recognition by the immune system.

In a preferred example, a large pool of immunoreactive T cells that are present or may be generated that are capable of reacting with these inert/naturally non-existent epitopes, but are not utilized and are considered "wasteful" because these epitopes are not being generated normally. This allows access to the pools of unused T cells for specifically directed therapeutic benefits. Again, computer-driven algorithms are run. Upon identification of the peptide sequences involved with antibody-binding interaction, these peptides are used to immunize animals and/or patients to generate specific antibodies that bind the protein and generate these novel peptides for subsequent immune recognition and response.

In another embodiment of the invention the method includes binding antibody or antibodies to the protein molecules and/or polypeptide regions and subjecting these bound and unbound/native complexes to ex vivo or in vitro to all forms of the proteasome machinery. Proteolytic digestion or cleavage products are obtained to observe differential yield of peptides for use in vaccine development. However, there is a drawback to this method in that it is not fully representative of all "mechanisms and proteolytic activities" that may occur in vivo within the cell itself, thereby limiting application. Such an approach however, is useful for the proof of concept in systems where it provides clean reproducible and predictable results, e.g. using various individual components of proteasomes and combinations thereof and then observing peptide patterns by mass spectroscopy analysis. It is also useful for the definition of peptide patterns generated in the presence and/or absence of proteasome inhibitors with and without bound antibody or antibodies.

Another embodiment of the method of the present invention involves application of this approach for identification of patient-specific treatment. Optimal antibody choices are made for the patient based upon patient's personal pre-existing immuno-reactive precursors to the AGIE/ABIE. First, PBMC (peripheral blood mononuclear cells) are obtained from patient and culture in presence or absence of cytokines (eg IL-2, IL-7, and IL-15) in the presence of FCS (fetal calf serum) or autologous serum. After optimal culture and expansion during a set number of days, the cells are tested in cytotoxicity assays against tumor cell lines that have been pre-incubated in the presence and absence of specific antibodies or antibody combination(s). Datasets from responses seen specifically to presence of antibodies and those where individual antibodies do not yield a response, but the combination that do are noted. The epitopes/vaccine peptides predicted or defined by the binding-sites of the antibodies are tested for fine reactivities in determination of which vaccines to use on patients.

Another embodiment of the method of the invention includes identifying antigens that are not expressed on the cell surface or for parts of protein that are always cytoplasmic or for proteins that are completely intracellular/intranuclear or cytoplasmic (e.g. p53, telomerase, etc.). Methods for expressing the "modulating" antibody or antibodies as endogenous proteins, so that they are able to bind these proteins intracellularly and modulate their processing and presentation from within. The endogenous expression methods included recombinant DNA expression systems as well as viral/bacterial vector delivery and expression systems.

For "shuttling" in the antibodies or single-chain antigen-binding fragment may be encapsulated within various delivery systems to include liposomes, micelles, nanoparticles and others.

Generation of "protective" or "suppressive" antibody preparations or combination capable of being administered passively for treatment of disease similar to the gamma-globulin shots were generated as polyclonal preps or as specific combinations of selected antibodies against one or more tumor or tissue specific antigens for that particular disease or condition.

The present invention is not restricted to HLA-A2 or class I peptides because one or more longer-length peptides from a known region or sequence can be used for the generation of AGIE/ABIE by the antibody or antibodies in question. The benefit is not having to be restricted by only specific or limited HLA types (Class I and II) in terms of patients that can be treated. Furthermore, although the majority of Class I and Class II peptides are derived from the processing of endogenous and exogenous proteins respectively, the well described and accepted mechanisms of "cross-presentation " allows for the presentation of all sources or peptides on both classes of MHC molecules.

In another embodiment of the invention, the method is used for identifying inducible vaccine responses by "discovering" the MAb/s that will generate AGIE/ABIE. This is achieved by first screening 10-mer or 20-mer consecutive or overlapping peptides from antigen for the highest precursor T-cell responses present in cancer and/or normal individuals. These "ultra-immunogenic" peptides are then used for generation of MAb in mice. These MAbs are then tested for ability to generate AGIE/ABIE specific responses in MAb-treated targets. MAbs that are promising are then humanized for therapeutic purposes. To ensure involvement of T cell specific immune responses the promising Fab' fragments are first used to eliminate purely antibody-dependent cell-mediated cytotoxicity (ADCC)-mediated activity.

Another way of specifically identifying peptides capable of generating antibodies that induce AGIE/ABIE that are most protective is by the method of screening serum sample from high-risk cancer individuals (those with family history of cancer, e.g. smokers who do and do not get lung cancer, patients who are 'cured') who do and do not develop cancer (or from progressor vs non-progressors in the case of AIDS) to look for Ab responses against the overlapping peptides from specific well characterized or defined tumor antigens (HER2/neu, prostate specific antigen or PSA, prostate specific membrane antigen or PSMA, Tyrosinase, melanoma Ags, etc.) that are present in the protected individuals or present in fully recovered/cured cancer patients. Based on the specific Ab responses that are found to be uniquely present or predominantly present in the 'protected' individuals, peptides are targeted for generation or 'discovering' of MAb that generated AGIE/ABIE. All descriptions herein were done for both Class I and Class II epitopes and responses.

The present invention provides enhanced lytic activity of antibody-treated tumor cells by vaccines identified from the binding sites of these antibodies. The present invention also provides a novel indication for antibodies already in clinical use for cancer treatment as combination therapy furthering development of vaccine treatment discovery.

## Claims

1. A method of identifying an immunogenic region of a self-tumor associated protein for use in modulation of the immune response for the treatment of cancer in combination with an antibody comprising the steps of:
identifying one or more peptide sequences of a self-tumor associated protein that are involved with binding to the antibody;
subjecting the one or more peptide sequences to an algorithm that identifies sequences suspected of being immunogenic;
screening all peptide fragments from the one or more peptide sequences; and
identifying an immunogenic peptide of the protein fragment, wherein the immunogenic peptide is for use in the treatment of cancer in combination with the antibody.

2. The method of claim 1, wherein the antibody-binding comprises polyclonal antibody binding.

3. The method of claim 1, wherein the antibody-binding comprises monoclonal antibody binding.

4. The method of any preceding claim, wherein the self-protein is a HER-2/neu protein.

5. The method of claim 1, wherein the step of identifying one or more peptide sequences of a self-protein that are involved with antibody-binding comprises:
binding an antibody to the self-protein forming a complex;
subjecting the complex to proteasome digestion; and
obtaining digestion products comprising one or more peptide sequences.

## Patentansprüche

1. Verfahren zur Identifizierung einer immunogenen Region eines körpereigenen Tumor-assoziierten Proteins zur Verwendung in der Modulation der Immunantwort bei der Behandlung von Krebs in Kombination mit einem Antikörper umfassend die Schritte:
Identifizierung einer oder mehrerer Peptidsequenzen eines körpereigenen Tumor-assoziierten Proteins, die an der Bindung an den Antikörper beteiligt sind;
Anwendung eines Algorithmus, der Sequenzen identifiziert, von denen vermutet wird, dass sie immunogen sind, auf die eine oder mehrere Peptidsequenzen;
Überprüfung aller Peptidfragmente der einen oder mehreren Peptidsequenzen; und Identifizierung eines immunogenen Peptids des Proteinfragments, wobei das immunogene Peptid zur Verwendung in der Behandlung von Krebs in Kombination mit dem Antikörper bestimmt ist.

2. Verfahren nach Anspruch 1, wobei die Antikörperbindung die Bindung eines polyklonalen Antikörpers umfasst.

3. Verfahren nach Anspruch 1, wobei die Antikörperbindung die Bindung eines monoklonalen Antikörpers umfasst.

4. Verfahren nach einem der vorangegangenen Ansprüche, wobei das körpereigene Protein ein HER-2/neu Protein ist.

5. Verfahren nach Anspruch 1, wobei der Schritt des Identifizierens einer oder mehrerer Peptidsequenzen eines körpereigenen Proteins, die an der Antikörperbindung beteiligt sind, umfasst:
Bindung eines Antikörpers an das körpereigene Protein, wodurch ein Komplex gebildet wird;
Unterziehen des Komplexes einer Proteasom-Verdauung; und
Erhalten von Verdauungsprodukten umfassend eine oder mehrere Peptidsequenzen.

## Revendications

1. Procédé d'identification d'une région immunogène d'une auto-protéine associée à une tumeur pour une utilisation dans la modulation de la réponse immunitaire pour le traitement du cancer en combinaison avec un anticorps comprenant les étapes suivantes:
identification d'une ou plusieurs séquences peptidiques d'une auto-protéine associée à une tumeur qui sont impliquées dans la liaison à l'anticorps;
soumission d'une ou plusieurs séquences peptidiques à un algorithme qui identifie des séquences suspectées d'être immunogènes;
criblage de tous les fragments peptidiques d'une ou plusieurs séquences peptidiques, et
identification d'un peptide immunogène du fragment de protéine, dans lequel le peptide immunogène est destiné à une utilisation dans le traitement du cancer en combinaison avec l'anticorps.

2. Procédé selon la revendication 1, dans lequel la liaison à l'anticorps comprend une liaison à un anticorps polyclonal.

3. Procédé selon la revendication 1, dans lequel la liaison à l'anticorps comprend une liaison à un anticorps monoclonal.

4. Procédé selon une quelconque revendication précédente, dans lequel l'auto-protéine est une protéine HER-2/neu.

5. Procédé selon la revendication 1, dans lequel l'étape d'identification d'une ou plusieurs séquences peptidiques d'une auto-protéine qui sont impliquées dans la liaison à un anticorps comprend:
la liaison d'un anticorps à l'auto-protéine formant un complexe;
la soumission du complexe à une digestion de protéasome; et
l'obtention de produits de digestion comprenant une ou plusieurs séquences peptidiques.
